Europäisches Patentamt
European Patent Office
Office européen des brevets

(11) Publication number: 0 169 969 A2

# (12) EUROPEAN PATENT APPLICATION

(21) Application number: 85103157.5

(22) Date of filing: 19.03.85

(51) Int. Cl.[4]: C 07 C 161/00
C 07 D 277/48, C 07 D 285/08
C 07 C 157/14, A 61 K 31/41
A 61 K 31/155

(30) Priority: 27.07.84 GB 8419223

(43) Date of publication of application:
05.02.86 Bulletin 86/6

(84) Designated Contracting States:
AT BE CH DE FR GB IT LI LU NL SE

(71) Applicant: HOECHST U.K. LIMITED
Hoechst House Salisbury Road
Hounslow Middlesex TW4 6JH(GB)

(72) Inventor: Cousins, Simon John
1 Gleman Close Greenleys
Milton Keynes Buckinghamshire(GB)

(72) Inventor: Michael, Jeffrey Daniel
5 Wood Street Woburn Sands
Milton Keynes Buckinghamshire(GB)

(72) Inventor: Ross, Barry Clive, Dr.
41 Blandford Avenue
Luton Bedfordshire(GB)

(74) Representative: Beck, Bernhard et al,
HOECHST AKTIENGESELLSCHAFT Zentrale
Patentabteilung Postfach 80 03 20
D-6230 Frankfurt/Main 80(DE)

## (54) Sulphamoylguanidine derivatives.

(57) Described are compounds of formula XLI

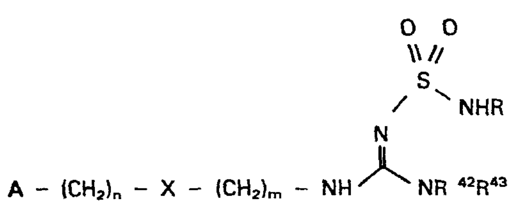

XLI

with R, $R^{42}$ and $R^{43}$ being hydrogen, alkyl, phenyl or phenalkyl A being phenyl, furyl, thiazolyl or thiadiazolyl; X being oxygen or sulphur; n being 0 or 1; and m being 2, 3 or 4.

Described is a process for their production reacting compounds XLIII

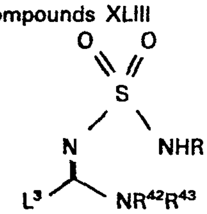

XLIII

(L being X-alkyl or X-phenylalkyl with compound XLV

$$A-(CH_2)_n-X-(CH_2)_m-NH_2 \qquad \text{XLV}$$

or reacting compound XLIV

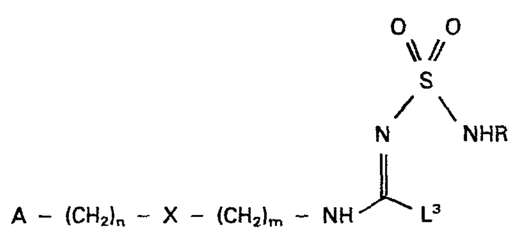

XLIV

with $HNR^{42}R^{43}$ XLVI.

Compounds XLI are valuable H-2 antagonists.

EP 0 169 969 A2

The present invention relates to sulphamoylguanidine derivatives which have histamine H-2 antagonist activity. The invention also relates to processes for the preparation of these derivatives and their salts, to pharmaceutical preparations comprising them, and to their use.

Histamine is one of a number of naturally occurring physiologically active substances which are thought to interact with specific receptors. In the case of histamine, there are at least two types: one is called the H-1 receptor (Ash and Schild, Brit. J. Pharmac. 1966, 27, 427), and the other is called the H-2 receptor (Black *et al* Nature 1972, 236, 385). The action of histamine at the H-1 receptor, for example, stimulation of bronchial and gastro-intestinal smooth muscle, is blocked by the compounds generally known as "antihistamines", but which are now also called histamine H-1 antagonists, for example, mepyramine. The action of histamine at the H-2 receptors, for example, stimulation of gastric acid secretion and heart rate, is not blocked by mepyramine but is blocked by other compounds, for example, burimamide and cimetidine.

Histamine H-2 antagonists may be used to treat those conditions resulting from stimulation by histamine of H-2 receptors, either alone, for example, in inhibiting gastric acid secretion and thus treating its sequelae, for example, gastric and peptic ulcers; or together with H-1 antagonists, for example, in allergic and certain inflammatory conditions.

The present invention provides compounds of formula XLI, which are histamine H-2 antagonists:

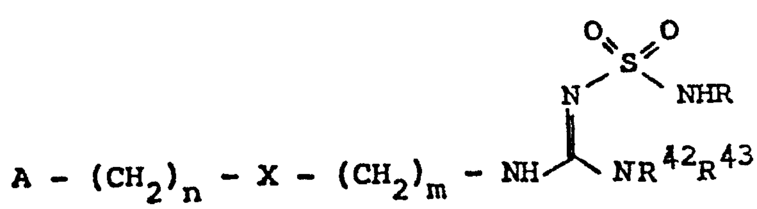

XLI

in which formula

R, $R^{42}$ and $R^{43}$, which may be the same or different, each represents a hydrogen atom, a straight or branched chain alkyl group having from 1 to 4 carbon atoms, a phenyl group or a phenalkyl group, the alkyl moiety of which has a straight or branched chain and from 1 to 4 carbon atoms, an alkyl, phenyl or phenalkyl group being unsubstituted or substituted by one or more substitutents, which may be the same or different, for example, by one or two substitutents, especially by one substitutent, selected from halogen atoms, straight and branched chain alkyl groups having from 1 to 4 carbon atoms, straight and branched chain alkoxy groups having from 1 to 4 carbon atoms, nitro and trifluoromethyl groups;

A represents a phenyl or furyl radical, which radical may be unsubstituted or may have one or more, especially one or two, substitutents which may be the same or different, selected from guanidino and mono-alkyl guanidino groups, the alkyl moiety of which has a straight or branched chain and from 1 to 4 carbon atoms, and from groups of the formula II

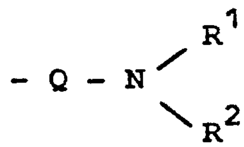

II

in which Q represents an alkylene group having from 1 to 4 carbon atoms, and $R^1$ and $R^2$, which may be the same or different, each represents a hydrogen atom, a straight or branched chain alkyl group having from 1 to 4 carbon atoms or, together with the nitrogen atom to which they are attached, $R^1$ and $R^2$ form a 4 to 8-membered ring which may contain an oxygen atom or another nitrogen atom, which nitrogen atom may have an alkyl group having from 1 to 4 carbon atoms as substituent, or

A represents a thiazolyl or thiadiazolyl radical that is substituted by one or more, especially by one or two, substituents which may be the same or different and which are as defined above for substitutents of phenyl and furyl groups A;

X represents an oxygen or sulphur atom;

n represents 0 or 1; and

m represents 2, 3 or 4.

Compounds of formula XLI in which one, two or three, especially one, of the radicals R, $R^{42}$ and $R^{43}$ represent(s) methyl groups and the others represent hydrogen atoms are preferred. Especially preferred are those compounds in which R , $R^{42}$ and $R^{43}$ each represents a hydrogen atom.

The present invention also provides salts of compounds of formula XLI, especially physiologically tolerable salts, for example, a salt with hydrochloric, sulphuric, hydrobromic, succinic, tartaric or maleic acid. The invention further provides hydrates of formula XLI.

It will be understood that the structure given above for formula XLI is only one of several possible tautomeric and isomeric representations. The present invention includes all tautomeric and isomeric forms of compounds of the general formula XLI.

The term "lower" is used in the present specification to denote a group, radical or molecule having up to 4 carbon atoms. Any aliphatic, especially alkyl group in the present specification may have a straight or branched chain.

In a group of formula II, when $R^1$ and $R^2$ form a 4 to 8-membered ring which may contain an oxygen atom or another nitrogen atom, which nitrogen atom may have an alkyl group having from 1 to 4 carbon atoms as substitutent, such a ring is, for example, a pyrrolidine, piperidine, morpholine or N-methylpiperazine ring.

When A represents a thiazolyl or thiadiazolyl group then, independently, n preferably represents the

integer 1, m preferably represents the integer 2, and X preferably represents a sulphur atom. Particularly preferred are those compounds in which A represents a thiazolyl or thiadiazolyl group, n represents the integer 1, m represents the integer 2 and X represents a sulphur atom. A preferred substituent for a compound in which A represents a thiazolyl or thiadiazolyl group is an unsubstituted or substituted guanidino group. Such a substitutent is preferably present on a ring carbon atom, and is preferably in the 2-position on a thiazol-4-yl ring or on the 5-position of a 1,2,4-thiadiazol-3-yl ring.

When A represents a furyl group then n preferably represents the integer 1, m preferably represents the integer 2, and X preferably represents a sulphur atom, either independently or, preferably, in combination. When A represents a furyl group, a preferred substitutent is a group of the general formula II, in which Q preferably represents a methylene group, and $R^1$ and $R^2$ both represent lower alkyl groups, especially methyl groups. A substituent on a 5-furyl ring is preferably in the 2-position.

When A represents a phenyl group then preferably n represents 0, m represents the integer 3, and X represents an oxygen atom, either independently or, preferably, in combination. When A represents a phenyl group, a preferred substitutent is a group of the general formula II in which Q preferably represents a methylene group and $R^1$ and $R^2$ both represent lower alkyl groups, or especially, methyl groups or, together with the nitrogen atom, form a ring as defined above, especially a piperidine ring. When a group of formula II is one of two substitutents on a phenyl group A, the group of formula II is preferably in the meta position relative to the other group.

The present invention also provides a process for the production of a compound of formula XLI, which comprises a) reacting a compound of formula XLIII

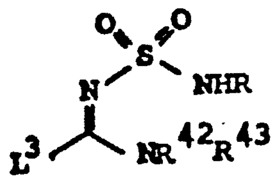

XLIII

in which R, $R^{42}$ and $R^{43}$ are as defined above, and $L^3$ represents a group $-XR^{46}$ in which X is as defined above and $R^{46}$ represents a straight or branched chain alkyl group having from 1 to 4 carbon atoms, an unsubstituted or substituted phenyl group, or an unsubstituted or substituted phenalkyl group having from 1 to 4 carbon atoms in the alkyl moiety, wherein a phenyl or phenalkyl group may be substituted by one or two, and especially by one substitutent, selected from alkyl, alkoxy, methylenedioxy, phenoxy, halogen, dialkylaminoalkyl (especially dimethylaminomethyl), trifluoromethyl, nitro, cyano, sulphonic acid, sulphonamide, amino, and mono- and di-alkylamino groups, an alkyl moiety having a straight or branched chain and from 1 to 4 carbon atoms, with a compound of the general formula XLV

$$A - (CH_2)_n - X - (CH_2)_m - NH_2 \qquad XLV$$

in which A, X, n and m are as defined above; or

b) reacting a compound of the general formula XLIV

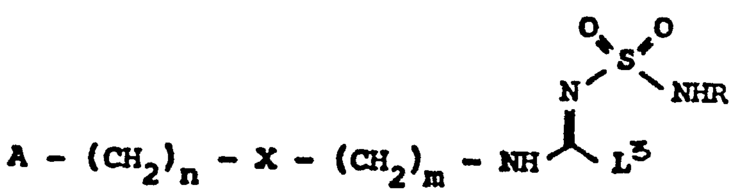

XLIV

in which A, n, m, X, R and $L^3$ are as defined above, with a compound of the general formula XLVI

XLVI

in which $R^{42}$ and $R^{43}$ are as defined above.

A phenyl or phenalkyl group $R^{46}$ may be substituted especially by one or more substituents, epecially by one

substitutent, selected from straight and branched chain alkyl groups having from 1 to 4 carbon atoms, especially methyl groups, halogen atoms, straight and branched chain alkoxy groups having from 1 to 4 carbon atoms, especially methoxy groups, and nitro groups.

In a group $L^3$, when X represents a sulphur atom, $R^{46}$ preferably represents a lower alkyl group, especially a methyl group, and when X represents an oxygen atom, $R^{46}$ preferably represents a phenyl group, especially a substituted phenyl group, for example, a mono-substituted phenyl group, especially a tolyl group or a nitrophenyl group, for example, an ortho-nitrophenyl group.

The reaction between compounds XLIII and XLV, and between compounds XLIV and XLVI, respectively, is generally carried out in a solvent, for example, an organic solvent, for example, a lower alcohol, for example, methanol, ethanol or isopropanol, dimethylformamide, tetrahydrofuran or acetonitrile, or in a mixture of two or more of such solvents. The reaction is generally carried out at a temperature within the range of from 5 to 75°C.

Compounds of the general formula XLV are known, and are described in the following: Belgian Patent Specifications 867 106 and 875 846; US Patent 4 128 658, British Patent Applications 2 001 624A and 2 084 581A, and European Patent Application 0 006 679.

A compound of formula XLV is reacted in the form of the free base, as shown. If it is initially present in the form of an acid addition salt, for example, as the hydrochloride or hydrobromide, this is converted into the free base during or, preferably, before reaction with compound XLIII (or compound XLVII as described below). Conversion is carried out with an organic or inorganic base, for example, sodium hydroxide or potassium hydroxide, or a tertiary amine, for example, triethylamine.

A compound of formula XLIV may be prepared by

reacting a compound of the general formula XLVII

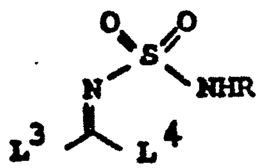

XLVII

in which R is as defined above, and $L^3$ and $L^4$ each represents a group $-X-R^{46}$ in which X and $R^{46}$ are as defined above, with the proviso that in any pair of leaving groups $L^3$ and $L^4$, the two radicals $R^{46}$ may be the same or different, but the two radicals X must be the same, with a compound of the general formula XLV, as defined above. Preferably compounds XLVII and XLV are used in equimolar amounts.

Similarly, a compound of formula XLIII may be prepared by reacting a compound of formula XLVII as defined above with a compound of formula XLVI as defined above, preferably in equimolar amounts.

The reaction between compounds XLVII and XLV, and between compounds XLVII and XLVI, is generally carried out under the same conditions as described above for the reaction between compounds XLIII and XLV and between compounds XLIV and XLVI.

As indicated above, the two leaving groups $L^3$ and $L^4$ may be the same or different, provided that X is the same in both cases. When X in $L^3$ and $L^4$ represents a sulphur atom, one group $R^{46}$ preferably represents a lower alkyl group, especially a methyl group, and the other group $R^{46}$ preferably represents a phenyl group; when X represents an oxygen atom, both groups $R^{46}$ preferably represent substituted phenyl groups, especially substituted by the preferred groups described above, for example, nitrophenyl groups.

It is not always necessary to isolate a resulting compound of formula XLIII or XLIV from the reaction mixture, and the conversion of either of these compounds

to a compound of formula XLI as described above may be carried out *in situ*. The present invention includes this method of producing a compound of formula XLI.

Compounds of formula XLVII in which R represents a hydrogen atom and $L^3$ and $L^4$ both represent alkylthio or both represent alkoxy groups are disclosed in UK Patent Specification No. 1 398 426, which discloses a process for the production of those compounds of formula XLVII in which R represents a hydrogen atom, and $L^3$ and $L^4$ both represent alkylthio groups only. Our co-pending application no.                    describes other compounds of formula XLVII and also describes a different process which enables all compounds of formula XLVII to be produced.

According to this process, a compound of the general formula XLVIII

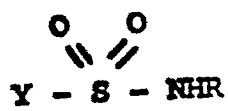

XLVIII

in which Y represents a halogen atom, especially a chlorine atom, and R is as defined above, is reacted with a compound of the general formula XXXIII or with a compound of the general formula XXXIV

XXXIII 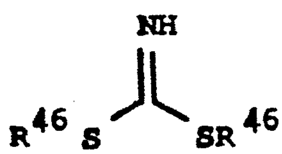

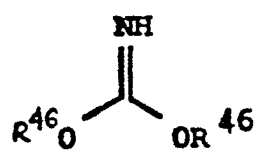 XXXIV

in which $R^{46}$ is as defined above.

A compound of formula XLVIII may be prepared as described in Chem. Ber. 91, 1339, (1958), in DE-OS 937,645, J. Org. Chem. 41 4028 (1976) or in J. Med. Chem. 15 (5) 538 (1972), and is preferably used when freshly prepared. A compound of formula XXXIII may be prepared as described in Z. Chem. 8 (12) 459-460 (1968), Bull. inst. recherches biol unic. Perm, 6, (10), 517-522, or Beilstein E113 page 156, and a compound of formula XXXIV as

described in Liebig's Annalen. 287 310, 319 and 321 (1895).

In a compound of formula XXXIII, one radical $R^{46}$ preferably represents a methyl group and the other preferably represents a phenyl group. In a compound of formula XXXIV, both radicals $R^{46}$ preferably represent substituted phenyl groups, for example, ortho-nitrophenyl groups.

The reaction between the compound of formula XLVIII and the compound of formula XXXIII or XXXIV is preferably carried out in the presence of an organic base, especially a tertiary amino base, for example, triethylamine.

The reaction is preferably carried out in the presence of an aprotic solvent, for example, acetonitrile, or an ether, an aromatic hydrocarbon, a chlorinated aliphatic or aromatic hydrocarbon, for example, diethyl ether, tetrahydrofuran, benzene or dichloromethane.

The reaction is preferably carried out under an inert atmosphere, for example, under nitrogen. The reaction is generally carried out at a temperature within the range of from -50 to +80°C, peferably from -5 to +45°C.

Preferably, a compound of formula XLVIII, advantageously freshly prepared, is added dropwise to a solution of the compound of formula XXXIII or XXXIV, and the base is preferably added simultaneously with the compound of formula II. This addition may be carried out over a period of from 5 minutes to 8 hours, preferably from 10 minutes to 2 hours.

As indicated above, the compounds of the present invention have histamine H-2 antagonist activity. Accordingly, the present invention provides a pharmaceutical preparation which comprises a compound of the general formula XLIor a physiologically tolerable salt thereof as active ingredient, in admixture or conjunction with a pharmaceutically suitable carrier. The preparation may be in a form suitable for enteral or parenteral administration, for example, for oral or intravenous

administration. The preparation may be in unit dosage form, for example, as tablets or capsules, or in unit or multiple dose ampoules or vials. From 0.1 to 10 mg of the active substance may be administered per kg body weight. A preparation of the invention may also comprise one or more pharmaceutically active substances, for example, histamine H-1 antagonists.

The present invention also provides a compound of the general formula I for use as a medicament, especially for use as a histamine H-2 antagonist, and further provides a method of treating conditions resulting from stimulation by histamine of H-2 receptors, either alone, for example, in inhibiting gastric acid secretion and thus treating its sequelae, for example, gastric and peptic ulcers, or together with H-1 antagonists, for example, in treating allergic and certain inflammatory conditions.

Examples of preferred compounds of formula XLI are the following:

(1) R = hydrogen, $R^{42}$ = hydrogen, $R^{43}$ = methyl, A = 3-(N,N-dimethylaminomethyl)phenyl, n = 0, X = O and m = 3 i.e.
N-[3-[3-(N,N-Dimethylaminomethyl)phenoxy]-n-propyl]-N'-methyl-N"-sulphamoylguanidine

(2) R = hydrogen, $R^{42}$ = hydrogen, $R^{43}$ = methyl, A = 3-piperidinylmethylphenyl, n = 0, X = O and m = 3 i.e.
N-[3-[3-(1-Piperidinylmethyl)phenoxy]-n-propyl]-N'-methyl-N"-sulphamoylguanidine

(3) R = $R^{42}$ = $R^{43}$ = hydrogen, A = 3-piperidinylmethylphenyl, n = 0, X = O and m = 3 i.e.
N-[3-[3-(1-Piperidinylmethyl)phenoxy]-n-propyl]-N'-sulphamoylguanidine

(4) R = $R^{42}$ = $R^{43}$ = hydrogen, A = 5-guanidino-3-(1,2,4-thiadiazolyl), n = 1, X = S and m = 2 i.e.
N-[2-[(5-Guanidino-1,2,4-thiadiazol-3-yl)methylthio]ethyl]-N'-sulphamoylguanidine

(5) R = hydrogen, $R^{42}$ = methyl, $R^{43}$ = hydrogen, A =

5-guanidino-3-(1,2,4-thiadiazolyl), n = 1, X = S and m = 2 i.e.

N-[2-[(5-Guanidino-1,2,4-thiadiazol-3-yl)methylthio]ethyl]-N'-methyl-N"-sulphamoylguanidine

(6) R = methyl, $R^{42}$ = $R^{43}$ = hydrogen, A = 2-guanidino-4-thiazolyl, n = 1, X = S and m = 2 i.e.

N-[2-[(2-Guanidino-4-thiazolyl)methylthio]ethyl]-N'-(N-methylsulphamoyl)guanidine

(7) R = hydrogen, $R^{42}$ = $R^{43}$ = methyl, A = 2-guanidino-4-thiazolyl, n = 1, X = S and m = 2 i.e.

N-[2-[(2-Guanidino-4-thiazolyl)methylthio]ethyl]-N'-dimethyl-N"-sulphamoylguanidine

and especially

(8) R = hydrogen, $R^{42}$ = hydrogen, $R^{43}$ = methyl, A = 2-guanidino-4-thiazolyl, n = 1, X = S and m = 2 i.e.

N-[2-[(2-Guanidino-4-thiazolyl)methylthio]ethyl]-N-methyl-N"-sulphamoylguanidine

and

(9) R = $R^{42}$ = $R^{43}$ = hydrogen, A = 2-guanidino-4-thiazolyl, n = 1, X = S and m = 2 i.e.

N-[2-[(2-Guanidino-4-thiazolyl)methylthio]ethyl]-N'-sulphamoylguanidine.

The following Examples are given by way of illustration only, and are not limiting.

EXAMPLE 1

N-[3-[3-(N,N-Dimethylaminomethyl)phenoxy]-n-propyl]-N'-methyl-N"-sulphamoylguanidine

Anhydrous monomethylamine gas was passed for 10 minutes through a solution of 420mg of N-[3-[3-(N,N-dimethylaminomethyl)phenoxy]-n-propyl]-S-methyl-N'-sulphamoylisothiourea in 10 ml of dry methanol. Cooling was required to maintain the reaction temperature at 20°C. After 24 hours, the solution was evaporated to dryness *in vacuo*, and the resulting semi-solid was triturated with ether and filtered to give 246mg of N-[3-[3-(N,N-dimethylaminomethyl)phenoxy]-*n*-propyl]-N'-methyl-N"-sulphamoylguanidine as a colourless solid, melting point 88-89°C.

$^1$H n.m.r. DMSO-$d_6$ 7.20 (1H, dd); 6.85 - 6.73 (5H, m, 2H exch.); 6.04 (2H, s, exch.); 3.98 (2H, t); 3.32 (2H, s); 3.25 (2H, dt); 2.67 (3H, d); 2.11 (6H, s); 1.93 (2H, m)

EXAMPLE 2

N-[3-[3-(N,N-Dimethylaminomethyl)phenoxy]-n-propyl]-S-methyl-N'-sulphamoylisothiourea

A solution containing 416mg of 3-[3-(N,N-dimethylaminomethyl)phenoxy]propylamine in 10 ml of dry ethanol was added dropwise to a stirred solution of 524mg of methyl,phenyl-N-sulphamoylimidodithiocarbonate in 20 ml of dry ethanol. The reaction mixture was stirred at room temperature for 16 hours, concentrated under reduced pressure and the residue purified by silica gel chromatography using a mixture of chloroform and methanol as eluant to provide 435 mg of N-[3-[3-(N,N-dimethylaminomethyl)phenoxy]-n-propyl]-S-methyl-N'-sulphamoyl-isothiourea as a colourless oil.

$^1$H n.m.r. 250MHz, $CDCl_3$, : 7.21 (1H, dd); 6.98 (1H, s); 6.84 (2H, m); 5.22 (1H, hump, exch.); 4.09 (2H, t); 3.54

(2H, t); 3.39 (2H, s); 2.39 (3H, s); 2.23 (6H, s); 2.11 (2H, m).

EXAMPLE 3

N-[2-[(2-Guanidino-4-thiazolyl)methylthio]ethyl]-S-methyl-N'-sulphamoylisothiourea

A solution of 2.31 g of 2-[(2-guanidino-4-thiazolyl)methylthio]ethylamine in 75 ml of ethanol was added over a period of 15 minutes to a stirred solution of 2.0 g of dimethyl N-sulphamoylimidodithiocarbonate in 50 ml of acetonitrile. The solution was heated at 60°C. for 4 hours, kept at room temperature for 16 hours, evaporated to dryness in vacuo, and purified by silica gel chromatography using a mixture of chloroform and methanol as eluant, to provide 2.73 g of N-[2-[[2-guanidino-4-thiazolyl]methylthio]ethyl]-S-methyl-N'-sulphamoylisothiourea as a white foam.

$^1H$ n.m.r., 250MHz, DMSO-$d_6$, : 6.70 - 6.10 (7H, br. humps, exch.); 6.52 (1H, s); 3.62 (2H, s); 3.40 (2H, m); 2.66 (2H, t); 2.31 (3H, br.s).

EXAMPLE 4

N-[2-[(2-Guanidino-4-thiazolyl)methylthio]ethyl]-S-methyl-N'-sulphamoylisothiourea

The above compound was prepared by a method analogous to that described in Example 2, using 1 molar equivalent of methyl,phenyl-N-sulphamoylimidodithiocarbonate and 1 molar equivalent of 2-[(2-guanidino-4-thiazolyl)methylthio]-ethylamine. Its properties are as described in Example 3.

EXAMPLE 5

N-[2-[(2-Guanidino-4-thiazolyl)methylthio]ethyl]-N-methyl-N"-sulphamoylguanidine

Monomethylamine gas was bubbled through a solution of 1.5g of N-[2-[(2-guanidino-4-thiazolyl)methylthio]ethyl]-S-methyl-N'-sulphamoylisothiourea in 20ml of anhydrous methanol for 15 minutes with cooling. The resuting mixture

was kept at room temperature overnight, then evaporated to dryness to give a semi-solid, which was purified by column chromatograpy using silica gel. Elution with chloroform/methanol mixtures gave the title compound: 993mg, m.p. 91-93°C.

$^1$H n.m.r. 250MHz DMSO-$d_6$ : 6.87 (4H, hump, exch.); 6.76 (2H, hump, exch.); 6.57 (1H, s); 6.07 (2H, s, exch.); 3.62 (2H, s); 3.28 (2H, dt); 2.69 (3H, d); 2.60 (2H, t).

EXAMPLE 6

N-[2-[(2-Guanidino-4-thiazolyl)methylthio]ethyl]-O-(2-nitrophenyl)-N'-sulphamoylisourea

To a solution of 2.5g of bis-(2-nitrophenyl)sulphamoylimidocarbonate in 50ml of dimethylformamide a solution of 1.5g of 2-[(2-guanidino-4-thiazolyl)methylthio]ethylamine in 25ml of dimethylformamide was added dropwise over 1 hour. After a further hour, the mixture was evaporated to dryness and the residue treated with chloroform, filtered and the filtrate chromatographed on Kieselgel 60 to give 470mg of the title compound on elution with chloroform/methanol mixtures. Crystallisation from chloroform/methanol mixtures gave pale yellow needles: m.p. 136-138°C

$^1$H nm.r. 250MHz DMSO-$d_6$ :2.73 (2H, t); 2.54 (2H, m); 3.64 (2H, s); 6.48 (1H, s); 6.66 (2H, s, exch.); 6.90 (4H, hump, exch.); 7.54 (2H, m); 7.85 (1H, m); 7.97 (1H, t); 8.19 (1H, m).

EXAMPLE 7

N-[2-[(2-Guanidino-4-thiazolyl)methylthio]ethyl]-N'-sulphamoylguanidine

Anhydrous ammonia gas was bubbled through a solution of 150mg of N-[2-[(2-guanidino-4-thiazolyl)methylthio]ethyl]-O-(2-nitrophenyl)-N'-sulphamoylisourea in a mixture of 50ml of dry acetonitrile and 10ml of dry methanol for 2 to 3 minutes. The solution became bright yellow. After 1 hour at room temperature, the mixture was evaporated to dryness

and the residue chromatographed on silica gel. Elution with chloroform removed the o-nitrophenol and with mixtures of chloroform, methanol and $NH_4OH$ (saturated solution) gave the title compound as a colourless solid: 80mg, m.p. 143-145°C.

$^1H$ n.m.r. DMSO-$d_6$ : 6.89 (4H, hump, exch.); 6.71 (1H, hump, exch.); 6.57 (1H, s); 6.53 (2H, hump, exch.); 6.10 (2H, s, exch.); 3.62 (2H, s); 3.24 ( 2H, m); 2.56 (2H, t)

EXAMPLE 8

N-[3-[3-(1-Piperidinylmethyl)phenoxy]-n-propyl]-N'-methyl-N"-sulphamoylguanidine

Monomethylamine gas was passed through a solution of 200mg of N-[3-[3-(1-piperidinylmethyl)phenoxy]-n-propyl]-S-methyl-N'-sulphamoylisothiourea in 15ml of methanol for 15 minutes. After a further 16 hours, the reaction mixture was evaporated to dryness and the residue chromatographed on silica gel with methylene chloride/ethanol/$NH_4OH$ (saturated solution) to give 103mg of the title compound as a thick colourless oil.

$^1H$ n.m.r. 250 MHz DMSO-$d_6$ : 7.21 (1H, dd); 6.75-6.86 (5H, m, 2H, exch.); 6.07 (2H, s, exch.); 3.99 (2H, t); 3.38 (2H, t); 3. 28 (2H, m); 2.69 (3H, d); 2.31 (4H, br. s); 1.94 (2H, m); 1.47 (4H, hump); 1.38 (2H, hump).

EXAMPLE 9

N-[3-[3-(1-Piperidinylmethyl)phenoxy]-n-propyl]-S-methyl-N'-sulphamoylisothiourea

50ml of an acetonitrile solution containing 520mg of S-methyl,S-phenyl N-sulphamoylimidodithiocarbonate and 500mg of 3-[3-(1-piperidinylmethyl)phenoxy]propylamine was kept at room temperature for 24 hours. After this time, the solution was evaporated to dryness and chromatographed on silica gel, eluting with chloroform/methanol mixtures to give 475mg of the title compound as an oil.

$^1H$ n.m.r. 250 MHz DMSO-$d_6$ : 7.22 (1H, dd); 6.83

(3H, m): 4.01 (2H, t); 3.44 (2H, m); 3.39 (2H, s); 2.32 (7H, br. s); 2.01 (2H, m); 1.49 (4H, br. s); 1.38 (2H, br. s).

EXAMPLE 10

N-[3-[3-(1-Piperidinylmethyl)phenoxy]-n-propyl]-N'-sulphamoylguanidine oxalate

To a solution of 954mg of bis-(2-nitropenoxy)sulphamoyl-imidocarbonate in 25ml of acetonitrile a solution of 744mg of 3-[3-(1-piperidinylmethyl)phenoxy]propylamine in 10ml of acetonitrile was added dropwise at room temperature over 15 minutes. The bright yellow solution was then treated with ammonia gas which was bubbled through the solution for 1-2 minutes. The bright orange solid which separated out (2-nitrophenol) was filtered off and discarded. The filtrate was chromatograped on silica gel, eluting with chloroform/methanol mixtures, to give 435mg of the free base of the title compound as a colourless glass.

This product was dissolved in ethanol and treated with 110mg of oxalic acid in ethanol. A white solid was precipitated, which was crystallised from ethanol and dried over silica gel: 394mg, m.p. 75-77°C.
$^1$H n.m.r. 250 MHz DMSO-$d_6$ : 7.35 (1H, dd); 6.99-7.10 (3H, m); 6.85 (1H, hump, exch.); 6.58 (2H, hump, exch); 6.09 (2H, hump, exch); 4.15 (2H, s); 4.01 (2H, t); 3.21 (2H, m); 3.00 (4H, br.s); 1.90 (2H, m); 1.70 (4H, br.s); 1.51 (2H, br.s).

EXAMPLE 11

N-[2-[(5-Guanidino-1,2,4-thiadiazol-3-yl)methylthio]ethyl]-N'-sulphamoylguanidine

2-[(5-Guanidino-1,2,4-thidiazol-3-yl)methylthio]ethylamine and bis-(2-nitrophenyl)-N-sulphamoylimidocarbonate were reacted and subsequently treated with ammonia by a procedure analogous to that described in Example 10 to give the title compound as a buff coloured foam.

$^1$H n.m.r. 250 MHz DMSO-$d_6$ : 7.16 (5H, s, exch.); 7.02 and 6.70 (1H, 2 humps, exch.); 6.53 (1H, hump, exch.); 6.11 (2H, s, exch.); 3.71 (2H, s); 3.30 (2H, m); 2.69 (2H, m).

EXAMPLE 12

N-[2-[(5-Guanidino-1,2,4-thiadiazoyl)methylthio]ethy]-N'-methyl-N"-sulphamoylguanidine

The above compound was obtained by a process analogous to that described in Example 11, but using monomethylamine in place of ammonia: colourless solid, m.p. 178-180°C.

$^1$H n.m.r. 250 MHz DMSO-$d_6$ : 7.15 (4H, s, exch.); 6.88 (1H, m, exch.); 6.75 (1H, m, exch.); 6.06 (2H, s, exch.); 3.71 (2H, s); 3.31 (2H, m); 2.69 (5H, m).

EXAMPLE 13

N-[2-[(2-Guanidino-4-thiazoyl)methylthio]ethy]-N'-(N-methylsulphamoyl)guanidine

196mg of N-[2-[(2-guanidino-4-thiazolyl)methylthio]ethyl]-N'-(N-methylsulphamoyl)-O-(2-nitrophenyl)-isourea was dissolved in 10ml of dry tetrahydrofuran, and 500mg of anhydrous ammonia dissolved in 10ml of dry tetrahydrofuran was added to give an orange solution. After a further hour at room temperature, the reaction mixture was evaporated to dryness and the residual oil chromatographed on silica gel with chloroform/methanol mixtures. The product was obtained as colourless foam after drying under vacuum: 130mg.

$^1$H n.m.r. 250 MHz DMSO-$d_6$ : 2.39 (3H, d); 2.55 (2H, t); 3.25 (2H, m); 3.60 (3H, d); 6.11 (1H, m); 6.56 1H, s); 6.64-6.97 (6H, br.m).

EXAMPLE 14

N-[2-[(2-Guanidino-4-thiazoyl)methylthio]ethy]-N'-(N-methylsulphamoyl)-O-(2-nitrophenyl)-isourea

604mg of 2-guanidino-4-[(2-aminomethyl)thiomethyl]-thiazole.2HCl was treated with a solution of 224mg of

potassium hydroxide in 5ml of anhydrous ethanol. The resulting suspension was stirred for a few minutes, filtered and the filtrate treated with a suspension of bis-(2-nitrophenoxy)-N-(N'-methylsulphamoyl)imidocarbonate in 10ml of ethanol. After 3 hours, the homogeneous solution was evaporated and the residual oil chromatographed on silica using chloroform/methanol mixtures as eluant. The product was crystallised from ethanol: 404mg, m.p. 90-91°C.

$^1H$ n.m.r. 250MHz DMSO-$d_6$ :2.30 (3H, d); 2.76 (2H, t); 3.44 (2H, m); 3.65 (2H, s); 6.47 (1H, s); 6.84 (4H, br.s); 7.53 (2H, m); 7.84 (1H, m); 7.96 (1H, t); 8.19 (1H, m).

EXAMPLE 15

N-[2-[(2-Guanidino-4-thiazolyl)methylthio]ethyl]-N'-dimethyl-N"-sulphamoylguanidine

Dimethylamine gas was bubbled through a solution of 250 mg of N-[2-[(2-guanidino-4-thiazolyl)methylthio]ethyl]-S-methyl-N'-sulphamoylisothiourea in 10 ml ethanol/acetonitrile (1:1) for 15 minutes with cooling. The reaction mixture was kept at room temperature for 48 hours then evaporated to dryness and the residue purified by chromatogaphy on silica gel. Elution with chloroform/methanol mixtures gave 174 mg of the title compound as a colourless foam.

$^1H$ nmr 250MHz DMSO-$d_6$ : 6.85 (5H, hump, exch); 6.53 (1H, s); 6.04 (2H, s, exch); 3.62 (2H, s); 3.36 (2H, m); 2.88 (6H, s); 2.64 (2H, t).

EXAMPLE 16

Bis-methyl-N-sulphamoyldithioimidocarbonate

To a solution of 3.75g of dithioimidocarbonate dimethyl ester in 100ml of dry benzene, 4.35 ul of triethylamine and a solution of 3.58g of freshly prepared sulphamoyl chloride in 40ml of dry benzene were added dropwise simultaneously at 18°C, with vigorous stirring under an atmosphere of

nitrogen. The addition was carried out over 30 minutes. After a further period of one hour at room temperature, the mixture was evaporated to dryness and triturated with chloroform. The resulting solid was filtered off and dried over silica gel: 3.37g m.p. 148-150°C. m/e 200.

EXAMPLE 17

Methyl,phenyl-N-sulphamoyldithioimidocarbonate

To a solution of 8.69g of methyl,phenyl-dithioimidocarbonate in 175ml of dry benzene, 8.14ml of triethylamine and a solution of 6.67g of freshly prepared sulphamoyl chloride in 80ml of dry benzene were added dropwise simultaneously at 18°C with vigorous stirring under an atmosphere of nitrogen. The addition was carried out over a period of one hour. External cooling was applied to maintain the temperature of 18°C. After a further 16 hours at room temperature, 100ml of water and 100ml of diethyl ether were added and the mixture was stirred until all the solids had dissolved. The layers were then separated and the organic phase washed with water, dried over $MgSO_4$, and then evaporated to give an oily solid which on trituration with benzene/hexane gave 4.5g of a colourless solid. Crystallisation from chloroform gave needles, m.p. 125-126°C. m/e 262

$^1H$ nmr 60MHz $CDCl_3$ : 2.40 (3H, s); 5.35 (2H, s. exch); 7.57 (5H, m).

EXAMPLE 18

Bis-(2-nitrophenyl)-N-sulphamoylimidocarbonate

To a vigorously stirred solution of 15.0g of bis-(2-nitrophenyl)-imidocarbonate in a mixture of 200ml of dry benzene, 100ml of ether and 25ml of acetonitrile at 40°C, 7.6ml of triethylamine and a solution of 5.7g of freshly prepared sulphamoyl chloride in 70ml of dry benzene were added dropwise simultaneously over a period of 45 minutes. After one hour at room temperature, 100ml of water and 100ml of ethyl acetate were added to dissolve any solids

0169969

present. The layers were then separated and the aqueous layer extracted with a further 100ml of ethyl acetate. The combined organic layers were washed with water, dried over $MgSO_4$, and then evaporated to give an oily solid, which on triturationwith ether gave 6.0g of a pale yellow solid, m.p. 165-167°C.

$^1H$ nmr 60MHz DMSO-$d_6$ : 4.35 (2H, hump, exch); 7.1-8.3 (8H, m).

EXAMPLE 19

Bis-(2-nitrophenyl)-N-(N'-methyl)sulphamoylimidocarbonate

6.06g of bis-(2-nitrophenyl)imidocarbonate was dissolved in 150ml of dry methylene dichloride. 2.84g of methylsulphamoyl chloride and 2.20g of triethylamine were added dropwise simultaneously to this solution with rapid stirring. After having been stirred for 15 minutes, the reaction solution was washed twice with water, dried over $MgSO_4$, and evaporated to a crystalline residue which was washed with ether and recrystallised from acetone/ether to give the title compound: 6.50g, m.p. 171-172°C.

$^1H$ nmr 60MHz DMSO-$d_6$ : 2.50 (3H, d), 7.1 - 8.3 (8H, m).

CLAIMS:

1. A compound of formula XLI

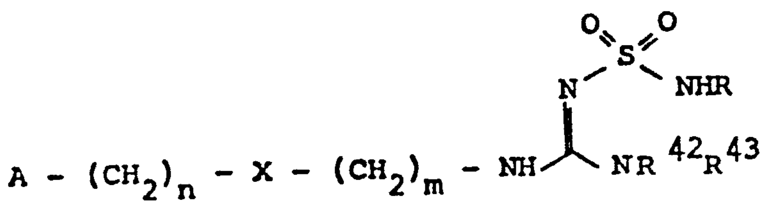

XLI

in which formula

R, $R^{42}$ and $R^{43}$, which may be the same or different, each represents a hydrogen atom, a straight or branched chain alkyl group having from 1 to 4 carbon atoms, a phenyl group or a phenalkyl group, the alkyl moiety of which has a straight or branched chain and from 1 to 4 carbon atoms,

wherein an alkyl, phenyl or phenyalkyl group may be unsubstituted or substituted by one or more substitutents, which may be the same or different, for example, by one or two substitutents, especially by one substitutent, selected from halogen atoms, straight and branched chain alkyl groups having from 1 to 4 carbon atoms, straight and branched chain alkoxy groups having from 1 to 4 carbon atoms, nitro and trifluoromethyl groups;

A represents a phenyl or furyl radical,

which radical may be unsubstituted or may have one or more, especially one or two, substitutents which may be the same or different, selected from guanidino and mono-alkyl guanidino groups, the alkyl moiety of which has a straight or branched chain and from 1 to 4 carbon atoms, and from groups of formula II

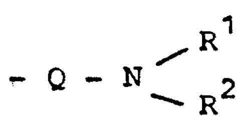

II

in which Q represents an alkylene group having from 1 to 4 carbon atoms, and $R^1$ and $R^2$, which may be the

same or different, each represents a hydrogen atom, or a straight or branched chain alkyl group having from 1 to 4 carbon atoms, or $R^1$ and $R^2$ together with the nitrogen atom to which they are attached form a 4 to 8-membered ring which may contain an oxygen atom or another nitrogen atom, which nitrogen atom may have an alkyl group having from 1 to 4 carbon atoms as substitutent, or

A represents a thiazolyl or thiadiazolyl radical that is substituted by one or more, especially by one or two, substituents which may be the same or different and which are as defined above for substitutents of phenyl and furyl groups A;

X represents an oxygen or sulphur atom;

n represents 0 or 1; and

m represents 2, 3 or 4;

and salts thereof.

2. A compound as claimed in claim 1, in which one, two or three, especially one, of the radicals R, $R^{42}$ and $R^{43}$ represent methyl groups and the others represent hydrogen atoms.

3. A compound as claimed in claim 1, in which R, $R^{42}$ and $R^{43}$ each represents a hydrogen atom.

4. A compound as claimed in any one of claims 1 to 3, A represents a thiazolyl or thiadiazolyl group, n represents the integer 1, m represents the integer 2, and X represents a sulphur atom, either independently or in combination.

5. A compound as claimed in claim 4, wherein A represents a thiazolyl or thiadiazolyl group substituted by an unsubstituted or substituted guanidino group.

6. A compound as claimed in claim 5, wherein a guanidino substitutent is present on a ring carbon atom, and is in the 2-position on a 4-thiazolyl ring orin the 5-position on a 1,2,4-thiadiazol-3-yl ring.

7. A compound as claimed in any one of claims 1 to 3, wherein A represents a furyl group, n represents the

integer 1, m represents the integer 2, and X represents a sulphur atom, either independently or in combination.

8.A compound as claimed in claim 7, wherein a furyl group A is substituted by a group of the general formula II, in which Q represents a methylene group, and $R^1$ and $R^2$ both represent lower alkyl groups.

9. A compound as claimed in any one of claims 1 to 3, wherein A represents a phenyl group, n represents 0, m represents the integer 3, and X represents an oxygen atom, either independently or in combination.

10. A compound as claimed in claim 9, wherein A represents a phenyl group substituted by a group of the general formula II in which Q represents a methylene group and $R^1$ and $R^2$ both represent lower alkyl groups or, together with the nitrogen atom, form a ring as defined above.

11. A compound as claimed in claim 10, wherein $R^1$ and $R^2$ both represent methyl groups or, together with the nitrogen atom, form a piperidine ring.

12. A compound as claimed in any one of claims 7 to 11, wherein a substituent on a phenyl group A is present in the meta-position, and a substituent on a 5-furyl group is in the 2-postion..

13. A compound as claimed in claim 1 wherein R= Hydrogen, $R^{42}$= hydrogen, $R^{43}$= methyl, A= 3-(N,N-dimethylaminomethyl)-phenyl, n= 0, X=0 and m= 3.

14. A compound as claimed in claim 1 wherein R= hydrogen, $R^{42}$= hydrogen, $R^{43}$= methyl, A= 3-piperidinylmethylphenyl, n=0, X= O and m= 3.

15. A compound as claimed in claim 1 wherein R = $R^{42}$ = $R^{43}$= hydrogen, A= 3-piperidinylmethylphenyl, n=0, X=0 and m=3.

16. A compound as claimed in claim 1 wherein R = $R^{42}$ = $R^{43}$= hydrogen, A= 2-guanidino-4-(1,2,4-thiadiazolyl), n= 1, X= S and m= 2.

17. A compound as claimed in claim 1 wherein R= hydrogen, $R^{42}$= methyl, $R^{43}$= hydrogen, A= 2-guanidino-4-(1,2,4-thiadiazolyl), n= 1, X= S and m= 2.

18. A compound as claimed in claim 1 wherein R= methyl, $R^{42}$ = $R^{43}$ = hydrogen, A= 2-guanidino-4-thiazolyl, n= 1, X= S and m= 2.

19. A compound as claimed in claim 1 wherein R= hydrogen, $R^{42}$ = $R^{43}$= methyl, A= 2-guanidino-4-thiazolyl, n= 1, X= S and m= 2.

20. A compound as claimed in claim 1 wherein R= hydrogen, $R^{42}$= hydrogen, $R^{43}$= methyl, A= 2-guanidino-4-thiazolyl, n= 1, X= S and m= 2.

21. A compound as claimed in claim 1 wherein R = $R^{42}$ = $R^{43}$= hydrogen, A= 2-guanidino-4-thiazolyl, n= 1, X= S and m= 2.

22. A process for the production of a compound of formula XLI as claimed in claim 1, which comprises

a) reacting a compound of formula XLIII

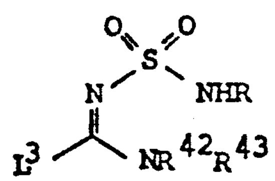

XLIII

in which R, $R^{42}$ and $R^{43}$ are as defined in claim 1, and $L^3$ represents a group $-XR^{46}$ in which X is as defined above and $R^{46}$ represents a straight or branched chain alkyl group having from 1 to 4 carbon atoms, an unsubstituted or substituted phenyl group, or an unsubstituted or substituted phenalkyl group having from 1 to 4 carbon atoms in the alkyl moiety, wherein a phenyl or phenalkyl group may be substituted by one or two, and especially by one substitutent, selected from alkyl, alkoxy, methylenedioxy, phenoxy, halogen, dialkylaminoalkyl (especially dimethylaminomethyl), trifluoromethyl, nitro, cyano, sulphonic acid, sulphonamide, amino, and mono- and di-alkylamino groups, an alkyl moiety having a straight or branched chain and from 1 to 4 carbon atoms, with a compound of the general formula XLV

$$A - (CH_2)_n - X - (CH_2)_m - NH_2 \qquad \text{XLV}$$

in which A, X, n and m are as defined in claim 1, or

b) reacting a compound of the general formula XLIV

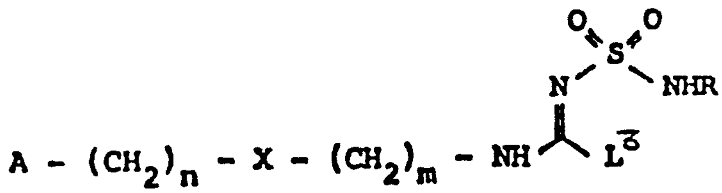

XLIV

in which A, n, m, X, R and $L^3$ are as defined above, with a compound of the general formula XLVI

$$HNR^{42}R^{43} \qquad XLVI$$

in which $R^{42}$ and $R^{43}$ are as defined above.

23. A process as claimed in claim 22, wherein in a group $L^3$, when X represents a sulphur atom, $R^{46}$ represents a lower alkyl group, especially a methyl group, and when X represents an oxygen atom, $R^{46}$ represents a phenyl group, especially a substituted phenyl group.

24. A process as claimed in claim 23, wherein a substituted phenyl group is a nitrophenyl group.

25. A process as claimed in any one of claims 22 to 24, wherein a compound of formula XLIV is prepared by reacting a compound of the general formula XLVII

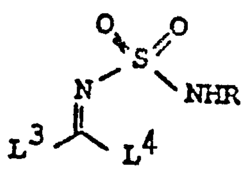

XLVII

in which R is as defined in claim 1 and $L^3$ and $L^4$ each represents a group $-X-R^{46}$ in which X and $R^{46}$ are as defined in claim 22, with the proviso that in any pair of leaving groups $L^3$ and $L^4$, the two radicals $R^{46}$ may be the same or different, but the two radicals X must be the same, with a compound of the general formula XLV, as defined in claim 22.

26. A process as claimed in any one of claims 22 to 25, wherein a compound of formula XLIII is prepared by reacting a compound of formula XLVII as defined in claim 25 with a compound of formula XLVI as defined in claim 22.

27. A pharmaceutical preparation which comprises a compound of the general formula XLI as claimed in any one of claims 1 to 21, or a physiologically tolerable salt thereof, in admixture or conjunction with a pharmaceutically suitable carrier.

28. A compound of the general formula XLI as claimed in any one of claims 1 to 21, or a physiologically tolerable salt thereof, for the manufacture of a medicament for the treatment of conditions resulting from stimulation by histamine of histamine H-2 receptors.

29. A method of treating conditions resulting from stimulation by histamine of histamine H-2 receptors in a mammal, which comprises administering to the mammal a compound of formula XLI as claimed in any one of claims 1 to 21.

30. A method as claimed in claim 29, wherein there is also administered to the mammal a histamine H-1 antagonist.

31. A compound of formula XLIV

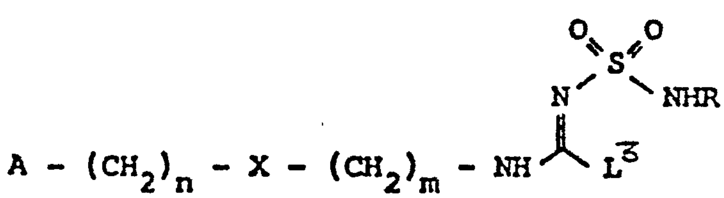

XLIV

in which A, n, m, X, R and $L^3$ are as defined in claim 22.

Claims for Austria:

1. A process for the production of a compound of formula XLI

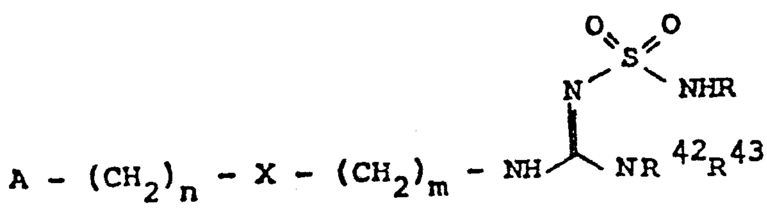

XLI

in which formula

R, $R^{42}$ and $R^{43}$, which may be the same or different, each represents a hydrogen atom, a straight or branched chain alkyl group having from 1 to 4 carbon atoms, a phenyl group or a phenalkyl group, the alkyl moiety of which has a straight or branched chain and from 1 to 4 carbon atoms,

wherein an alkyl, phenyl or phenyalkyl group may be unsubstituted or substituted by one or more substitutents, which may be the same or different, for example, by one or two substitutents, especially by one substitutent, selected from halogen atoms, straight and branched chain alkyl groups having from 1 to 4 carbon atoms, straight and branched chain alkoxy groups having from 1 to 4 carbon atoms, nitro and trifluoromethyl groups;

A represents a phenyl or furyl radical,

which radical may be unsubstituted or may have one or more, especially one or two, substitutents which may be the same or different, selected from guanidino and mono-alkyl guanidino groups, the alkyl moiety of which has a straight or branched chain and from 1 to 4 carbon atoms, and from groups of formula II

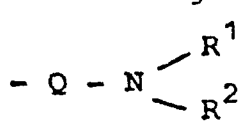

II

in which Q represents an alkylene group having from 1 to 4 carbon atoms, and $R^1$ and $R^2$, which may be the

same or different, each represents a hydrogen atom, or a straight or branched chain alkyl group having from 1 to 4 carbon atoms, or $R^1$ and $R^2$ together with the nitrogen atom to which they are attached form a 4 to 8-membered ring which may contain an oxygen atom or another nitrogen atom, which nitrogen atom may have an alkyl group having from 1 to 4 carbon atoms as substitutent, or

A represents a thiazolyl or thiadiazolyl radical that is substituted by one or more, especially by one or two, substituents which may be the same or different and which are as defined above for substitutents of phenyl and furyl groups A;

X represents an oxygen or sulphur atom;

n represents 0 or 1; and

m represents 2, 3 or 4;

and salts thereof, which comprises

a) reacting a compound of formula XLIII

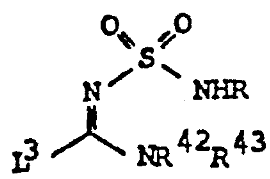

XLIII

in which R, $R^{42}$ and $R^{43}$ are as defined above and $L^3$ represents a group $-XR^{46}$ in which X is as defined above and $R^{46}$ represents a straight or branched chain alkyl group having from 1 to 4 carbon atoms, an unsubstituted or substituted phenyl group, or an unsubstituted or substituted phenalkyl group having from 1 to 4 carbon atoms in the alkyl moiety, wherein a phenyl or phenalkyl group may be substituted by one or two, and especially by one substitutent, selected from alkyl, alkoxy, methylenedioxy, phenoxy, halogen, dialkylaminoalkyl (especially dimethylaminomethyl), trifluoromethyl, nitro, cyano, sulphonic acid, sulphonamide, amino, and mono- and di-alkylamino groups, an alkyl moiety having a straight or branched chain and from 1 to 4 carbon atoms,

with a compound of the general formula XLV

$$A - (CH_2)_n - X - (CH_2)_m - NH_2 \qquad XLV$$

in which A, X, n and m are as defined above, or

b) reacting a compound of the general formula XLIV

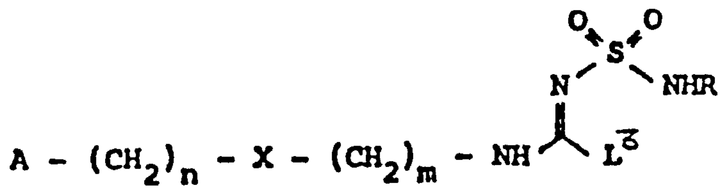

XLIV

in which A, n, m, X, R and $L^3$ are as defined above, with a compound of the general formula XLVI

$$HNR^{42}R^{43} \qquad XLVI$$

in which $R^{42}$ and $R^{43}$ are as defined above.

2. A process as claimed in claim 1, wherein in a group $L^3$, when X represents a sulphur atom, $R^{46}$ represents a lower alkyl group, especially a methyl group, and when X represents an oxygen atom, $R^{46}$ represents a phenyl group, especially a substituted phenyl group.

3. A process as claimed in claim 2, wherein a substituted phenyl group is a nitrophenyl group.

4. A process as claimed in any one of claims 1 to 3, wherein a compound of formula XLIV is prepared by reacting a compound of the general formula XLVII

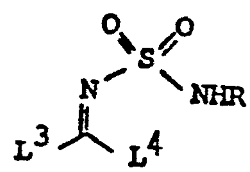

XLVII

in which R is as defined in claim 1 and $L^3$ and $L^4$ each represents a group $-X-R^{46}$ in which X and $R^{46}$ are as defined above with the proviso that in any pair of leaving groups $L^3$ and $L^4$, the two radicals $R^{46}$ may be the same or different, but the two radicals X must be the same, with a compound of the general formula XLV, as defined in claim 1.

5. A process as claimed in any one of claims 1 to 4, wherein a compound of formula XLIII is prepared by reacting a compound of formula XLVII as defined in claim 4, with a compound of formula XLVI as defined in claim 1.

6. A process according to claim 1, wherein a compound XLI is produced in which one, two or three, especially one, of the radicals R, $R^{42}$ and $R^{43}$ represent methyl groups and the others represent hydrogen atoms.

7. A process as claimed in claim 1, in which R, $R^{42}$ and $R^{43}$ each represents a hydrogen atom.

8. A process as claimed in claim 1, in which A represents a thiazolyl or thiadiazolyl group, n represents the integer 1, m represents the integer 2, and X represents a sulphur atom, either independently or in combination.

9. A process as claimed in claim 1, wherein A represents a thiazolyl or thiadiazolyl group substituted by an unsubstituted or substituted guanidino group.

10. A process as claimed in claim 1, wherein a guanidino substituent is present on a ring carbon atom, and is in the 2-position on a 4-thiazolyl ring or in the 5-position on a 1,2,4-thiadiazol-4-yl ring.

11. A process as claimed in claim 1, wherein A represents a furyl group, n represents the integer 1, m represents the integer 2, and X represents a sulphur atom, either independently or in combination.

12. A process as claimed in claim 1, wherein a furyl group A is substituted by a group of the general formula II, in which Q represents a methylene group, and $R^1$ and $R^2$ both represent lower alkyl groups.

13. A process as claimed in claim 1, wherein A represents a phenyl group, n represents 0, m represents the integer 3, and X represents an oxygen atom, either independently or in combination.

14. A process as claimed in claim 1, wherein A represents a phenyl group substituted by a group of the general formula II in which Q represents a methylene group and $R^1$ and $R^2$ both represent lower alkyl groups or, together with the nitrogen atom, form a ring as defined above.

15. A process as claimed in claim 14, wherein $R^1$ and $R^2$ both represent methyl groups or, together with the nitrogen atom, form a piperidine ring.

16. A process as claimed in claim 1, wherein a substituent on a phenyl group A is present in the meta-position, and a substituent on a 5-furyl group is in the 2-position.

17. A process as claimed in claim 1, wherein N-/3-/3-(N,N-Dimethylaminomethyl)phenoxy7-n-propyl7-N'-methyl-N"-sulphamoylguanidine is produced.

18. A process according to claim 1, wherein N-/3-/3-(1-Piperidinylmethyl)phenoxy7-n-propyl7-N'-methyl-N"-sulphamoyl-guanidine is produces.

19. A process according to claim 1, wherein N-/3-/3-(1-Piperidinylmethyl)phenoxy7-n-propyl7-N'-sulphamoylguanidine is produced.

20. A process according to claim 1, wherein N-/2-/(5-Guanidino-1,2,4-thiadiazol-3-yl)methylthio7ethyl7-N'-sulphamoylguanidine is produced.

21. A process according to claim 1 in which N-/2-/(5-Guanidino-1,2,4-thiadiazolyl)methylthio7ethyl7-N'-methyl-N"-sulphamoylguanidine is produced.

22. A process according to claim 1 in which N-/2-/(2-Guanidino-4-thiazolyl)methylthio7ethyl7-N'-(N-methylsul-phamoyl)guanidine is produced.

23. A process according to claim 1 in which N-/2-/(2-Guanidino-4-thiazolyl)7methylthio7ethyl7-N'-dimethyl-N"-sulphamoylguanidine is produced.

24. A process according to claim 1 in which N-/2-/(2-Guanidino-4-thiazolyl)methylthio7ethyl7-N-methyl-N"-sulphamoylguanidine is produced.

25. A process according to claim 1 in which N-/2-/(2-Guanidino-4-thiazolyl)methylthio7ethyl7-N'-sulphamoyl-guanidine is produced.

26. A process for the production of a pharmaceutical preparation which comprises admixing or conjoining a compound of the general formula XLI as produced according to claim 1, or a physiologically tolerable salt thereof, with a pharmaceutically suitable carrier.

27. A method of treating conditions resulting from stimulation by histamine of histamine H-2 receptors in a mammal, which comprises administering to the mammal a compound of formula XLI as produced according to claim 1.

28. A process according to claim 1 for the production of a compound of formula XLIV

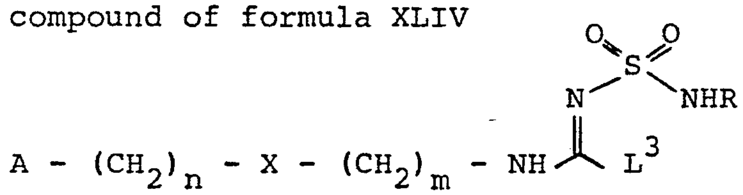

XLIV

in which A, n, m, X, R and $L^3$ are as defined in claim 1.